# FASCICULE DE BREVET EUROPEEN

(11) **EP 1 877 011 B1**
(45) Date de publication et mention de la délivrance du brevet: **27.08.2014**
(21) Numéro de dépôt: 06764584.6
(22) Date de dépôt: 02.05.2006
(51) Int. Cl.: A61F 2/44

(54) **PROTHESE DE DISQUE INTERVERTEBRAL**
BANDSCHEIBENPROTHESE
INTERVERTEBRAL DISC PROSTHESIS

(30) Priorité: 03.05.2005 FR 0504510
(43) Date de publication de la demande: 16.01.2008
(73) Titulaire: Spineart SA, 1215 Genève (CH)
(72) Inventeur: LEVIEUX, Jérôme, 1293 Bellevue (CH)
(74) Mandataire: Cabinet Plasseraud
(86) Numéro de dépôt international: PCT/FR2006/000982
(87) Numéro de publication internationale: WO 2006/117474

(56) Documents cités:
- DE-U1- 20 315 611
- DE-U1-202004 009 542
- US-A1- 2004 002 761

## Description

La présente invention se rapporte aux prothèses de disques intervertébraux.

Plus particulièrement, l'invention concerne une prothèse de disque intervertébrale comportant un plateau supérieur, un plateau inférieur présentant une surface d'appui sensiblement plane, un élément intermédiaire comportant une base présentant un pourtour et surmontée d'une calotte sphérique, c'est-à-dire un volume formant une saillie, le plateau supérieur présentant une empreinte complémentaire de la calotte sphérique et venant au contact de ladite calotte de manière à définir une liaison rotule entre le plateau supérieur et l'élément intermédiaire, la base de l'élément intermédiaire venant au contact de la surface d'appui, le plateau inférieur présentant un guide comprenant deux bords antérieur et postérieur se faisant face, présentant entre eux une distance sensiblement constante et maintenant entre eux l'élément intermédiaire, le pourtour de la base venant au contact d'au moins un des bords.

US 2004/0143332-A1 décrit une prothèse du type décrit précédemment dans laquelle l'élément intermédiaire se déplace selon une trajectoire en arc de cercle en glissant dans un rail.

Cependant les différents éléments constituant la prothèse peuvent subir des frottements localisés qui peuvent endommager le fonctionnement optimal de la prothèse à long terme.

DE 203 15 611 U concerne une prothèse de disque intervertébral, dont la calotte est maintenue de manière centrale par rapport à un évidement et est entourée d'amortisseurs. Seul est possible un déplacement d'avant en arrière (dit « antéropostérieur »), et sans possibilité de déplacement par roulement de l'élément intermédiaire le long des bords. Les bords avec lesquels l'élément intermédiaire est en contact sont les bords latéraux de la prothèse.

US-A-04/0002761 décrit une prothèse de disque intervertébral comportant un élément concave. Seul un déplacement antéropostérieur est possible et notamment pas un déplacement par roulement de l'élément intermédiaire le long des bords antérieur et postérieur de la prothèse.

La présente invention a notamment pour but de proposer une prothèse présentant une durée de vie améliorée par rapport aux prothèses de l'art antérieur. A cet effet, la présente invention a pour objet une prothèse de disque comme défini à la revendication 1. Selon l'invention, une prothèse de disque du genre en question est caractérisée en ce que le pourtour présente une forme circulaire et est adapté pour permettre le déplacement par roulement de l'élément intermédiaire le long des bords.

Grâce à ces dispositions, la prothèse ne présente plus de points d'usure localisés, en remplaçant le contact par frottement sur les bords du guide par un roulement, ce qui permet d'augmenter la durée d'utilisation optimale de la prothèse. Dans un exemple, les bords du guide présentent une forme de type conique non circulaire, de manière à guider l'élément intermédiaire selon une trajectoire de type conique.

Dans divers modes de réalisation de la prothèse selon l'invention, on peut éventuellement avoir recours en outre à l'une et/ou à l'autre des dispositions suivantes :
- l'élément intermédiaire est déplaçable, pour chaque point de la trajectoire, selon un axe perpendiculaire à une tangente au point de la trajectoire, d'une valeur comprise entre 0,1 et 3 mm entre les deux bords du guide;
- l'amplitude de déplacement de l'élément intermédiaire le long de la trajectoire est de 0,01 à 2 fois le diamètre de la base 22 ;
- le guide est un évidement réalisé dans le plateau inférieur ;
- la base de l'élément intermédiaire possède une collerette circulaire périphérique extérieure comportant plusieurs lumières, de manière à amortir les chocs entre le bords du guide et la collerette par déformation de ladite collerette ;
- la collerette est réalisée en matériau polyéthylène à haut poids moléculaire.

D'autres caractéristiques et avantages de l'invention apparaîtront au cours de la description suivante d'un de ses modes de réalisation, donné à titre d'exemple non limitatif, en regard des dessins joints.

Sur les dessins :
- la figure 1 représente une vue en coupe verticale de la prothèse selon l'invention disposée entre deux vertèbres ;
- la figure 2 représente une vue en élévation d'une partie d'une prothèse selon une première variante de réalisation ;
- la figure 3 représente une vue en élévation d'une partie de la prothèse selon une deuxième variante de réalisation ;
- la figure 4 représente une vue en élévation de l'élément intermédiaire selon une troisième variante de réalisation.

Sur les différentes figures, les mêmes références désignent des éléments identiques ou similaires.

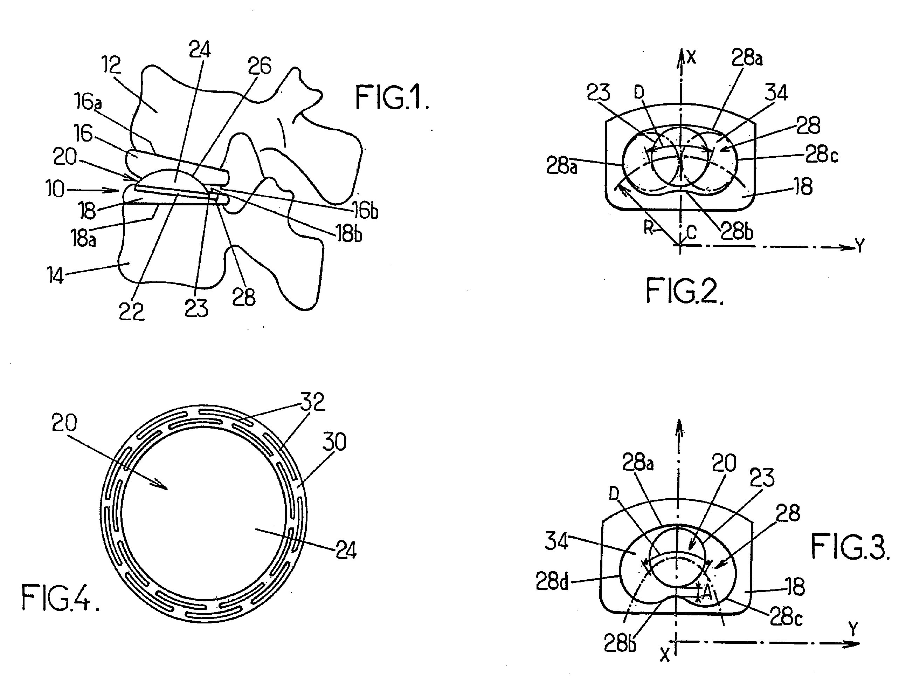

La présente invention se rapporte à une prothèse de disque 10 qui est destinée à être disposée entre deux vertèbres d'une colonne vertébrale 12, 14. Par exemple la figure 1 illustre une vue de profil de deux vertèbres 12, 14 d'une colonne vertébrale entre lesquelles est disposée la prothèse 10 selon l'invention. Ce type de prothèse 10 peut être utilisée pour remplacer un disque intervertébral sujet à des dégénérescences suite à un traumatisme, des maladies ou la vieillesse.

Ces dégénérescences peuvent entraîner une altération de l'espace naturel entre deux vertèbres. Le rétrécissement de cet espace naturel peut entraîner une pression qui s'exerce sur certains nerfs, et par conséquent des douleurs peuvent apparaître.

La prothèse de disque selon l'invention peut donc être utilisée pour maintenir l'espace naturel entre deux vertèbres.

Elle doit aussi permettre aux vertèbres de se déplacer l'une par rapport à l'autre selon un mouvement naturel. Notamment elle doit permettre un mouvement de rotation axial qui correspond en partie au mouvement de rotation du tronc ou du cou en cervical du corps humain, un mouvement antéropostérieur qui correspond à un mouvement de flexion ou d'extension de la partie supérieure du corps ou de la tête en cervical, et un mouvement latéral qui correspond à une inclinaison de la partie supérieure du corps, ou du cou en cervical.

La prothèse 10 de disque selon l'invention comporte un plateau supérieur 16 et un plateau inférieur 18 présentant chacun une face externe 16a, 18a orientée vers respectivement une vertèbre supérieure et un vertèbre inférieure. Sur chacune des faces externes 16a, 18a sont disposées des dents, non représentées, qui permettent l'ancrage des plateaux 16, 18 dans les vertèbres.

Les plateaux supérieur 16 et inférieur 18 comprennent aussi chacun une surface interne 16b, 18b. Les surfaces internes s'étendent latéralement selon un premier axe antéropostérieur X et s'étendant longitudinalement selon un deuxième axe Y perpendiculaire au premier axe, en vis à vis et elles sont en contact avec un élément intermédiaire 20.

L'élément intermédiaire 20 présente une base circulaire 22 présentant un pourtour 23 circulaire. La base 22 est surmontée d'une calotte sphérique supérieure 24. La base 22 est montée mobile sur la surface interne 18b du plateau inférieur 18, et la calotte sphérique 24 coopère avec une empreinte 26 réalisée dans la surface interne 16b du plateau supérieur 16. Cette empreinte 26 présente un profil sphérique, ce qui permet d'obtenir une liaison rotule entre la calotte sphérique 24 de l'élément intermédiaire 20 et le plateau supérieur 16.

Le plateau supérieur 16 est réalisé dans un matériau du type chrome - cobalt ou titane ou acier inoxydable et l'élément intermédiaire est réalisé dans un matériau plastique, de type polyéthylène haute densité, ayant de très bonnes caractéristiques de glissement.

La liaison rotule entre le plateau supérieur 16 fixé à la vertèbre supérieure 12 et l'élément intermédiaire 20 relié lui-même à la vertèbre inférieure 14 par le plateau inférieur 18 permet de reproduire les mouvements de flexion ou d'extension et les mouvements d'inclinaison entre deux vertèbres 12, 14 d'une colonne vertébrale.

La figure 3 représente une vue en élévation de la surface interne 18b du plateau inférieur 18 et de l'élément intermédiaire 20. La face interne 18b du plateau inférieur 18 présente un évidement 28 qui guide le déplacement de l'élément intermédiaire 20. Tel que représenté sur la figure 3, l'évidement 28 présente une forme de « haricot », délimité par une surface d'appui 34 sensiblement plane, un bord avant 28a, un bord arrière 28b et deux bords extrêmes 28c, 28d, reliant le bord avant 28a et le bord arrière 28b. Le bord avant 28a et le bord arrière 28b sont symétriques par rapport à l'axe antéropostérieur (X) et présentent chacun sensiblement une forme de type conique, illustrée à la figure 3 par une forme parabolique ouverte vers l'arrière selon l'axe antéropostérieur.

Il est aussi possible que les bords (28a, 28b, 28c, 28d) forment une succession de coniques.

Sur la figure 3, l'axe antéropostérieur X représenté est l'axe de symétrie de la trajectoire parabolique. La trajectoire parabolique est représentée par la ligne discontinue qui correspond à la ligne médiane entre les deux bords 28a, 28b.

Cette caractéristique permet à l'élément intermédiaire 20 de se déplacer selon une trajectoire de type conique telle qu'un arc de cercle, une portion de parabole, d'hyperbole ou d'ellipse, ou de type succession de conique, telle que plusieurs arcs de cercles adjacents, ce qui confère à l'élément intermédiaire un mouvement plus proche du mouvement réel de rotation entre deux vertèbres.

Sur la figure 2, la trajectoire représentée est un arc de cercle.

Sur la figure 3, la trajectoire représentée est une parabole, dont l'équation cartésienne réduite est du type y² = 2px.

Par ailleurs, l'amplitude de déplacement D de long de la trajectoire de type conique, entre deux bord extrêmes, est de l'ordre de 0,01 à 2 fois le diamètre de la base 22 de l'élément intermédiaire 20, de façon à répondre aux différents cas pathologiques et de permettre au patient de retrouver la capacité de réaliser un mouvement proche du mouvement réel.

Par ailleurs, l'évidement 28 est réalisé de manière à ce que l'élément intermédiaire 20 puisse translater de l'avant vers l'arrière afin d'obtenir une translation vers l'arrière de l'élément intermédiaire lors d'un mouvement de flexion, et une translation vers l'avant de l'élément intermédiaire 20 lors d'un mouvement d'extension.

L'amplitude A de ces mouvements de l'avant vers l'arrière, le long de la trajectoire, est comprise, suivant les cas pathologiques, avantageusement entre 0.1 et 3 mm, et de préférence entre 0,5 et 3mm, pour chaque point de la trajectoire, selon un axe perpendiculaire à une tangente au point de la trajectoire.

Cette caractéristique de l'évidement 28 permet d'obtenir une prothèse capable de reproduire une liaison intervertébrale naturelle notamment lors d'un mouvement de flexion et de rotation combiné.

Lorsque la base 22 de l'élément intermédiaire 20 se déplace dans l'évidement 28 réalisé sur la surface interne 18b du plateau inférieur 18, le pourtour 23 de la base 22 entre en contact avec les bords 28a, 28b de l'évidement 28. Le mouvement de l'élément intermédiaire 20 est alors obtenu par roulage de l'élément intermédiaire 20 le long des bords 28a, 28b délimitant l'évidement 28, ce qui évite des phénomènes d'usure prématurée dus aux frottements, comme lors d'un contact par glissement pur. Un glissement entre le fond de la base 22 et la surface d'appui de l'évidement 28 pourra être obtenu si la surface d'appui de l'évidement 28 est traité pour améliorer ses caractéristiques de glissement (par exemple un traitement polymiroir, ou revêtement de nitrure) et si le fond de la base est réalisé en polyéthylène à haut poids moléculaire, afin de limiter les frottements lors du déplacement de l'élément intermédiaire 20 et par conséquent une usure prématurée.

Par exemple si les bords de l'évidement présentent une forme en arc de cercle, chaque point du pourtour 23 de la base 22 décrira une courbe cycloïdale.

Selon une variante de l'invention représentée à la figure 4, la base de l'élément intermédiaire comporte une collerette circulaire 30 périphérique extérieure qui comporte plusieurs lumières 32 traversantes ou non, de manière à amortir les chocs entre les bords 28a, 28b de l'évidement 28 et la collerette 30. En effet, la collerette 30 est réalisée dans un matériau du type polyéthylène à haut poids moléculaire ou en tout autre matériau bio-compatible présentant de bonnes caractéristiques d'amortissement, qui avec la présence des lumières 32 présente une capacité de déformation suffisante pour amortir le contact lors de l'arrivée en butée de l'élément intermédiaire contre les bords 28a, 28b de l'évidement 28. Cela permet de limiter la douleur articulaire ressentie par le patient.

## Revendications

1. Prothèse de disque intervertébrale comportant :
- un plateau supérieur (16),
- un plateau inférieur (18) présentant une surface d'appui sensiblement plane,
- un élément intermédiaire comportant une base (22) présentant un pourtour (23) et surmontée d'une calotte sphérique (24),
le plateau supérieur présentant une empreinte complémentaire de la calotte sphérique et venant au contact de ladite calotte de manière à définir une liaison rotule entre le plateau supérieur (16) et l'élément intermédiaire (20),
la base de l'élément intermédiaire venant en contact de la surface d'appui,
le plateau inférieur (18) présentant un guide (28) comprenant deux bords antérieur et postérieur (28a, 28b) se faisant face
et maintenant entre eux l'élément intermédiaire,
le pourtour de la base de la calotte venant au contact d'au moins un des bords antérieur et postérieur (28a, 28b), le pourtour présentant une forme circulaire et étant adapté pour permettre le déplacement par roulement de l'élément intermédiaire le long des bords (28a, 28b), **caractérisé en ce que** lesdits bords antérieur et postérieur (28a, 28b) présentent entre eux une distance sensiblement constante, et **en ce que** lesdits bords (28a, 28b) présentent une forme parabolique, de manière à guider l'élément intermédiaire selon une trajectoire de forme parabolique.

2. Prothèse de disque selon l'une quelconque des revendications précédentes, dans laquelle l'élément intermédiaire (20) est déplaçable, pour chaque point de la trajectoire, selon un axe perpendiculaire à une tangente au point de la trajectoire, d'une valeur comprise entre 0,1 et 3 mm entre les deux bords (28a, 28b).

3. Prothèse de disque selon l'une quelconque des revendications précédentes, dans laquelle le pourtour circulaire présente un diamètre et l'amplitude de déplacement de l'élément intermédiaire le long de la trajectoire est de 0,01 à 2 fois le diamètre de la base (22).

4. Prothèse de disque selon l'une quelconque des revendications précédentes, dans laquelle le guide (28) est un évidement réalisé dans le plateau inférieur (18).

5. Prothèse de disque selon l'une quelconque des revendications 1 à 4, dans laquelle la base (22) de l'élément intermédiaire (20) possède une collerette (30) circulaire périphérique extérieure comportant plusieurs lumières (32), de manière à amortir les chocs entre le bords du guide (28) et la collerette (30) par déformation de ladite collerette.

6. Prothèse de disque selon la revendication précédente, dans laquelle la collerette (30) est réalisée en matériau polyéthylène à haut poids moléculaire.

## Patentansprüche

1. Bandscheibenprothese mit:
- einer oberen Platte (16),
- einer unteren Platte (18), die eine im Wesentlichen ebene Auflagefläche aufweist,
- einem Zwischenelement (20), das eine Basis (22) aufweist, die einen Umfangsrand (23) hat und von einer kugelförmigen Kalotte (24) überragt ist,
wobei die obere Platte eine Vertiefung aufweist, die komplementär zur kugelförmigen Kalotte ist und in Kontakt mit der Kalotte kommt, um eine Kugelgelenkverbindung zwischen der oberen Platte (16) und dem Zwischenelement (20) zu bilden,
wobei die Basis des Zwischenelements in Kontakt mit der Auflagefläche kommt,
wobei die untere Platte (18) eine Führung (28) aufweist, die einen vorderen und hinteren Rand (28a,28b) aufweist, die sich gegenüberliegen und zwischen sich das Zwischenelement halten,
wobei der Umfangsrand der Basis der Kalotte in Kontakt mit dem vorderen und/oder hinteren Rand (28a,28b) kommt,
wobei der Umfangsrand kreisförmig ist und eine Abrollbewegung des Zwischenelements entlang der Ränder (28a, 28b) erlaubt,
**dadurch gekennzeichnet, dass** der vordere und hintere Rand (28a,28b) zwischen sich einen im Wesentlichen konstanten Abstand haben, und dadurch, dass die Ränder (28a,28b) eine parabolische Form haben, um das Zwischenelement entlang einer parabelförmigen Trajektorie zu führen.

2. Bandscheibenprothese nach einem der vorstehenden Ansprüche, wobei das Zwischenelement (20) an jedem Punkt der Trajektorie entlang einer Achse, die senkrecht zu einer Tangente am Punkt der Trajektorie ist, um einen Betrag zwischen 0,1 und 3mm zwischen den beiden Rändern (28a,28b) verschiebbar ist.

3. Bandscheibenprothese nach einem der vorstehenden Ansprüche, wobei der kreisförmige Umfangsrand einen Durchmesser hat und die Amplitude der Verschiebung des Zwischenelements entlang der Trajektorie 0,01 bis 2 mal der Durchmesser der Basis (22) ist.

4. Bandscheibenprothese nach einem der vorstehenden Ansprüche, wobei die Führung (28) eine in der unteren Platte (18) gebildete Aussparung ist.

5. Bandscheibenprothese nach einem der Ansprüche 1 bis 4, wobei die Basis (22) des Zwischenelements (20) an ihrem Außenumfang einen kreisförmigen Kragen (30) mit mehreren Langlöchern (32) aufweist, um durch Verformung des Kragens die Stöße zwischen den Rändern der Führung (28) und dem Kragen (30) zu dämpfen.

6. Bandscheibenprothese nach dem vorstehenden Anspruch, wobei der Kragen (30) aus einem Polyethylenmaterial eines hohen Molekulargewichts hergestellt ist.

## Claims

1. Intervertebral disc prosthesis comprising:
- an upper plate (16),
- a lower plate (18) with a substantially flat support surface,
- an intermediate element with a base (22) which has a perimeter (23) and which is surmounted by a spherical cap (24),
the upper plate having an indentation which matches the spherical cap and which comes into contact with said cap so as to define a ball-and-socket joint between the upper plate (16) and the intermediate element (20),
the base of the intermediate element coming into contact with the support surface,
the lower plate (18) having a guide (28) with anterior and posterior edges (28a, 28b) facing each other and holding between them the intermediate element,
the perimeter of the base of the cap coming into contact with at least one of the anterior and posterior edges (28a, 28b),
the perimeter having a circular shape and being adapted to allow the intermediate element to move by rolling along the edges (28a, 28b),
**characterized in that** said anterior and posterior edges (28a, 28b) have between them a substantially constant distance, and **in that** said edges (28a, 28b) have a parabolic shape, in such a way as to guide the intermediate element along a trajectory of parabolic shape.

2. Disc prosthesis according to either one of the preceding claims, wherein the intermediate element (20) is displaceable, for each point of the trajectory, along an axis perpendicular to a tangent at the point of the trajectory, by a value of between 0.1 and 3 mm between the two edges (28a, 28b).

3. Disc prosthesis according to either one of the preceding claims, wherein the circular perimeter has a diameter, and the amplitude of movement of the intermediate element along the trajectory is from 0.01 to 2 times the diameter of the base (22).

4. Disc prosthesis according to any one of the preceding claims, wherein the guide (28) is a recess formed in the lower plate (18).

5. Disc prosthesis according to any one of Claims 1 to 4, wherein the base (22) of the intermediate element (20) has an outer peripheral circular flange (30) comprising several slots (32), so as to absorb the shocks between the edges of the guide (28) and the flange (30) by deformation of said flange.

6. Disc prosthesis according to the preceding claim, wherein the flange (30) is made of high molecular weight polyethylene.
